# EUROPEAN PATENT SPECIFICATION

(11) **EP 4 308 568 B1**
(45) Date of publication and mention of the grant of the patent: **14.10.2026**
(21) Application number: 22714811.1
(22) Date of filing: 14.03.2022
(51) Int. Cl.: C07D 487/04, C07D 495/04, A61P 1/00, A61K 31/519, A61P 11/00

(54) **HETEROCYCLIC DERIVATIVES AS JANUS KINASE INHIBITORS**
HETEROCYCLISCHE DERIVATE ALS JANUS-KINASEHEMMER
DÉRIVÉS HÉTÉROCYCLIQUES UTILISÉS EN TANT QU'INHIBITEURS DE JANUS KINASE

(30) Priority: 15.03.2021 EP 21162525
(43) Date of publication of application: 24.01.2024
(73) Proprietor: Chiesi Farmaceutici S.p.A., 43122 Parma (IT)
(72) Inventor: ACCETTA, Alessandro, 43122, Parma (IT); RANCATI, Fabio, 43122, Parma (IT); RIZZI, Andrea, 43122, Parma (IT); ZIHER, Dinko, 43122, Parma (IT); MESIC, Milan, 43122, Parma (IT); ELENKOV, Ivaylo, 43122, Parma (IT)
(74) Representative: Chiesi Farmaceutici S.p.A.
(86) International application number: PCT/EP2022/056552
(87) International publication number: WO 2022/194782

(56) References cited:
- EP-A1- 3 327 021
- WO-A1-02/096909
- WO-A1-2012/022265
- WO-A2-2006/069080
- CHOUGH CHIEYEON ET AL: "Development of selective inhibitors for the treatment of rheumatoid arthritis: (R)-3-(3-(Methyl(7H-pyrrolo[2,3-d]pyrimidin-4-yl)amino)pyrrolidin-1-yl)-3-oxopropanenitrile as a JAK1-selective inhibitor", BIOORGANIC, vol. 26, no. 8, 1 May 2018 (2018-05-01), AMSTERDAM, NL, pages 1495 - 1510, XP055923592, ISSN: 0968-0896, DOI: 10.1016/j.bmc.2018.01.021

## Description

### FIELD OF THE INVENTION

The present invention relates to chemical compounds useful as JAK inhibitors, such as JAK 1, useful for the treatment of various inflammatory disease including asthma, COPD and other respiratory diseases.

### BACKGROUND OF THE INVENTION

The JAK family consists of non-receptor tyrosine protein kinases and has four main members, JAK1, JAK2, JAK3, and TYK2. More than 50 cytokines and growth factors bind to type I and II receptors noncovalently associated with different combinations of JAK kinases. The signalling triggered by the ligands consists in tyrosine phosphorylation of receptors by JAK and recruitment of one or more STATs proteins. Tyrosine-phosphorylated STATs dimerize and are then transported into the nucleus through the nuclear membrane to regulate specific genes. JAKs have seven homology domains (the JAK homology domain, JH). Starting from the carboxyl terminus, JH1 is the first JH, known as the kinase domain, and is composed of approximately 250 amino acid residues. JH1 encodes a kinase protein that constitutes the kinase structure domain that phosphorylates a substrate; JH2 is a pseudokinase domain which regulates the activity of the kinase domain. JAK3 is expressed in the bone marrow and lymphatic system, as well as endothelial cells and vascular smooth muscle cells; other members are expressed in almost all tissues (Hu X et al., Signal Transduct Target Ther. 2021, 26;6(1):402). Many cellular processes are downstream JAK/STAT signalling: hematopoiesis, immune balance, tissue repair, inflammation, apoptosis, and adipogenesis. Different biological responses are regulated by specific pairing of JAK isoforms. JAK1/JAK3 combination mediates IL-2, -4, -7, -9, -15, and -21 signalling that is relevant for growth/maturation of lymphoid cells, differentiation/homeostasis of T-cells/NK cells, B-cell class switching and other inflammatory processes. Combinations of JAK1/TYK2-JAK1/JAK2, regulate the signal associated with the innate immune response, such as IL-6 and the type I interferons, involved into naive T cell differentiation, T cell homeostasis, granulopoiesis and other inflammatory processes. (Howell MD et al., Front. Immunol. 2019, 10, 2342). JAK2 frequently associates with itself (JAK2/ JAK2) controlling the signalling of various cytokines and growth factors, such as IL-3, IL-5, granulocyte macrophage colony-stimulating factor (GM-CSF), erythropoietin (EPO), and thrombopoietin (TPO) (Hodge et al., Clin Exp Rheumatol 2016; 34(2):318-28).

Genetically modified mouse models and human diseases prove the importance of JAK/STAT pathways in immune fitness. In particular, overexpression or mutations involving some JAK isoforms as well as aberrant JAK/STAT signalling drive malignancies of hematopoietic and lymphoid tissues as well as inflammatory disorders. Currently, several Food and Drug Administration (FDA)- and/or EU- approved JAK inhibitors are in clinical use. Two (ruxolitinib and fedratinib) small molecules are in use for hematologic disorders as myelofibrosis and polycythemia vera; six JAK inhibitors (tofacitinib, baricitinib, ruxololitinib, filgotinib, upadicitinib and delgocitinib in Japan) result in use for immune-mediated disorders as rheumatoid arthritis, polyarticular juvenile idiopathic arthritis, atopic dermatitis, ulcerative colitis and acute graft-versus-host disease. Moreover, some of these drugs as well as others are currently under phase II and III of clinical trials for indications that span from autoimmune diseases (lupus, vitiligo, etc.), inflammatory bowel disease to Non-Hodgkin lymphoma and COVID-19 (Hu X. et al., Sig Transduct Target Ther 2021, 6: 402).

The small molecules targeting JAK/STAT represent an attractive option also for the therapy of fibrotic disorders. In fact, inflammatory cytokines (IL-4, IL-3, IL-6, IL-11, IL-31, etc) and growth factors (FGF, VEGF, etc.) involved in the fibrotic processes activate JAK/STAT pathway. Ruxolitinib tested in a bleomycin-induced fibrosis mouse model ameliorated the fibrotic lesions in lung, and reduced levels of fibrotic molecular markers (Zhang, Y et al., Ann. Rheum. Dis. 2017, 76, 1467-1475) while tofacitinib acted as a preventive agent in experimental dermal and pulmonary fibrosis (Wang, W et al., Scleroderma Relat. Disord. 2020, 5, 40-50). In patients, some case reports were studied. A single-case report corroborated the efficacy and safety of tofacitinib in combination with nintedanib in the management of an aggressive interstitial lung disease with poor prognosis (Conca, W et al., Front. Pharmacol. 2020, 11, 5857619). Baricitinib was demonstrated to be a safe immune modulator that reduces the biomarkers' levels of lung fibrosis and inflammation in RA patients, including a subgroup with interstitial lung disease (D' Alessandro M et al., Int. Immunopharmacol. 2020, 86, 106748).

In COVID-19, there are some JAK inhibitors undergoing phase II/II clinical trials, and they are tofacitinib, baricitinib, and ruxolitinib. Baricitinib and ruxolitinib were associated with a reduced risk of mortality. They reduced the use of invasive mechanical ventilation and had a borderline impact on the admission rate of the intensive care unit and the incidence of acute respiratory distress syndrome (ARDS). (Wijaya, I. et al. Clin. Epidemiol. Glob. Health 2021, 11, 100755). Ruxolitinib also was tested in COVID-19 patients. and improved the clinical symptoms and chest computed tomography images (Cao Y. et al., J. Allergy Clin. Immunol. 2020 146, 137-146).

Asthma can be included in the plethora of immune-mediated diseases for which pathogenesis is characterized by an essential role of JAK/STAT signalling. Asthma is a chronic inflammatory disease of the airways due to a complex interplay between immune response, genetic susceptibility and nonspecific external stimuli like cold, allergens and exercise leading to hyperresponsiveness, remodelling of the airways, ultimately contributing to airflow limitation. Severe asthma affects 5% to 15% of the population with adult asthma (which is 300 million people worldwide) and represents a public health issue associated with increased mortality, increased hospitalizations, significant burden of symptoms, health care costs, and missed work and school (Steve NG et al., J Allergy Clin Immunol 2021;148:953-63). Severe asthma represents a subset of difficult-to-treat asthma and occurs in patients whose disease remains uncontrolled despite the use of high doses of inhaled corticosteroids (ICSs) combined with long-acting β-agonists or other controllers. To date, four types of biologics are licensed for severe asthma, i.e. omalizumab (anti-immunoglobulin E) antibody, mepolizumab and reslizumab (anti-interleukin [IL]-5antibody), benralizumab (anti-IL-5 receptor a antibody) and dupilumab (anti-IL-4 receptor alpha antibody). Despite their efficacy, many patients continue to experience exacerbations or uncontrolled disease, indicating a need for more novel therapies (Israel E, Reddel HK. N Engl J Med 2017; 377:965-76).

Recently, the better understanding of asthma pathobiology brought to a shift from a phenotypic classification system to the introduction of the "endotype" concept. According to the latter, classification is performed on the basis of pathophysiologic mechanisms and clinical biomarkers associated with a given patient (Wenzel SE et a., Am J Respir Crit Care Med 2021;203:809-21). There are two major endotypes in asthma: type 2 and non-type 2. The type 2 pathway is defined by activation of cytokines derived from TH2 cells and group 2 innate lymphoid cells (ILC2s); these include IL-4, IL-5, and IL-13 that cause airway inflammation by activating eosinophils, B cells, airway epithelial cells, and other cell types. Biomarkers of type 2 asthma include blood/sputum eosinophilia and elevated levels of fractional exhaled nitric oxide (FENO) and IgE. The type 2-low pathway is characterized by absence of type 2-high cytokines and biomarkers, and it manifests either increased levels of neutrophils in the airways or a paucigranulocytic profile, with normal levels of airway neutrophils and eosinophils. Type 2-low asthma is currently not well understood, and it likely encompasses multiple distinct endotypes. Potential mediators and/or biomarkers of T2 low endotypes under investigation include IL-6, IL-17 A/F, IL-23, Type I interferons, CXCL10, TNF, alarmins (TSLP, IL-25, IL-33), IL-1β, IL-8, IFN-γ (Hinks TSC et al., ERJ 2021, 57 (1) 2000528).

Almost all the mediators mentioned above both for T2 and T2-low endotypes activate JAK/STAT pathway, here the rationale for the potential use of JAK inhibitors in both endotypes of severe asthma. Targeting simultaneously several cytokines by JAK inhibitors may offer advantage over the biologics (for no-responder patients) and standard therapies (for patients who remain uncontrolled) considering their administration on top of ICS.

Despite strong rationale of JAK inhibitors in asthma, safety concerns may arise by administration of systemic inhibitors or may limits administration into particular asthma subjects such as children. Considering that Asthma is a lung restricted disease, inhalatory route of administration for a JAK inhibitor may offers the advantage of therapeutic efficacy while limiting systemic exposure and correlated side effects. To date, some companies are developing inhaled JAK inhibitor for asthma treatment. Astrazeneca pipeline include AZD-0449 (completed Phase I clinical trial) and AZD-4604 (ongoing Phase I clinical trial); Theravance Biopharma is starting a new preclinical program on TD-8236 inhaled JAK inhibitor and Kinaset/Vectura is developing VR588 (ongoing Phase I clinical trial) as inhalatory compound. Many preclinical studies sponsored by the companies mentioned above demonstrated the efficacy of JAK inhibitors in the modulation of asthma. In the preclinical phase of drug development, JAK1/3 inhibitor R256 (now referred as AZD0449) orally given showed be effective in decreasing airway resistance, BAL eosinophilia, mucus production and if administered during sensitization, also TH2 cytokine responses (Ashino S et al., J Allergy Clin Immunol 2014;133:1162-74). iJak-381 from Genentech given as dry powder reduced BAL eosinophilia, CCL11, airway resistance, and Muc5AC in OVA-challenged mice. Moreover, it reduced BAL eosinophilia, neutrophilia, CCL11, and CXCL1 in a in mouse model of chronic exposure to AAH allergens (Dengler HS et al., Sci Transl Med 2018;10:eaao2151). Moreover, an oral JAK inhibitor as Tofacitinib, formulated for being administered as aerosol, reduced eosinophils count in a house dust mite mouse model of asthma (Younis US et al., AAPS PharmSci-Tech 2019;20:167).

Another respiratory disease that could benefit from lung restricted JAK inhibition is Chronic obstructive pulmonary disease (COPD), an inflammatory disease of the lung, most commonly resulting from cigarette smoke exposure, characterised by a largely irreversible and progressive airflow limitation. Despite inflammatory cytokines are drivers of chronic airway inflammation and some of them trigger JAK/STAT activation (IL-6, IFN-γ, IL-2, etc.), the role of this pathway in COPD pathogenesis is poorly characterized.. Phosphorylated-STAT4+ cells (Di Stefano A et al., Eur Respir J. 2004 Jul; 24(1):78-85) were found to be increased in COPD compared to non-smokers healthy controls. In another study, phosphorylated-STAT3+ and phosphorylated-STAT1+ cells counts were higher in lung biopsies of COPD patients than non-smokers controls while it was not possible to reproduce previous data on phosphorylated-STAT4 molecule (Yew-Booth L et al., Eur Respir J 2015; 46(3):843-5). These data might also suggest a therapeutic use of JAK inhibitors also in COPD disease.
European patent application EP 3 327 021 A1; Chough C, et Al.; Bioorg Med Chem. 2018 May 1;26(8):1495-1510. doi: 10.1016/j.bmc.2018.01.021. Epub 2018 Feb 3. PMID: 29452839; international patent applications WO 2006/069080; WO 02/096909 and WO 2012/022265 are all documents disclosing inhibitors for JAK kinases differing from the compound of the present invention at least for the structural features of the pyrrolidine group and N-substituent therof.

In view of the number of pathological responses which are mediated by JAK enzymes, there is a continuing need for inhibitors of JAK enzymes which can be useful in the treatment of many disorders and particularly respiratory diseases.

Thus, the finding of novel and potent JAK inhibitor suitable for local administration to the lungs for treatment of asthma and respiratory disease still remain an important need.

### SUMMARY OF THE INVENTION

Accordingly, it is one object of the present invention to provide 3-amino pyrrolidine derivatives or a pharmaceutically-acceptable salt thereof, that are useful as JAK kinase inhibitors.

It is another object of the present invention to provided pharmaceutical compositions comprising such compounds, methods of using such compounds to treat respiratory diseases, and processes and intermediates useful for preparing such compounds.

### DETAILED DESCRIPTION OF THE INVENTION

### Definitions

The term "Pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts of the invention comprise ions of alkali or alkaline earth metals such as potassium, sodium, calcium or magnesium. Those obtained by reacting the main compound, functioning as a base, with an inorganic or organic acid to form a salt comprise, for example, salts of hydrochloric, hydrobromic, sulfuric, phosphoric, methane sulfonic, camphor sulfonic, acetic, oxalic, maleic, fumaric, succinic and citric acids.

Many organic compounds can form complexes with solvents in which they are reacted or from which they are precipitated or crystallized. These complexes are known as "solvates" which are a further object of the invention. Polymorphs and crystalline forms of compounds of formula (I), or of pharmaceutically acceptable salts, or solvates thereof are a further object of the invention.

Whenever basic amino or quaternary ammonium groups are present in the compounds of formula (I), physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkaline or alkaline earth metal ions.

Compounds of formula (I) when they contain one or more stereogenic center, may exist as optical stereoisomers.

Where the compounds of the invention have at least one stereogenic center, they may accordingly exist as enantiomers. Where the compounds of the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

"Single stereoisomer", "single diastereoisomer" or "single enantiomer", when reported near the chemical name of a compound indicate that the isomer was isolated as single diastereoisomer or enantiomer (e.g via chiral chromatography) but the absolute configuration at the relevant stereogenic center was not determined/assigned.

Atropisomers result from hindered rotation about single bonds where the steric strain barrier to rotation is high enough to allow for the isolation of the conformers (Bringmann G et al, Angew. Chemie Int. Ed. 44 (34), 5384-5427, 2005. doi:10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomer mixtures are included in the scope of the present invention.

The invention further concerns the corresponding deuterated derivatives of compounds of formula (I). In the context of the present invention, deuterated derivative means that at least one position occupied by a hydrogen atom is occupied by deuterium in an amount above its natural abundance. Preferably, the percent of deuterium at that position is at least 90%, more preferably at least 95%, even more preferably 99%.

All preferred groups or embodiments described above and here below for compounds of formula (I) may be combined among each other and apply as well *mutatis mutandis.*

As above mentioned, the present invention provides compounds of general formula (I), acting as JAK inhibitors, to processes for the preparation thereof, pharmaceutical compositions comprising them either alone or in combination with one or more active ingredient, in admixture with one or more pharmaceutically acceptable carriers.

In a first aspect of the present invention is to provide 3-aminopyrrolidine derivatives of formula I 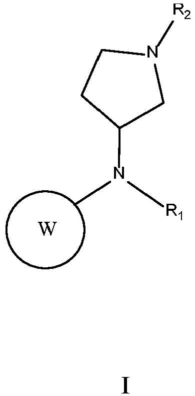 wherein,
R₁ is H or methyl;
R₂ is selected from a phenyl, phenylmethyl, pyridinyl and pyrimidinyl group substituted by one or more group selected from the group -CN, F, Cl, (thiazol-2-yl)aminocarbonyl, (methoxy)carbonyl, (hydroxy)carbonyl;
W is a bicyclic heteroaryl selected from J1 or J2: 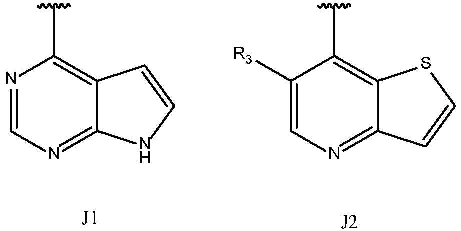
wherein R₃ is (ethoxy)carbonyl, (hydroxy)carbonyl or (amino)carbonyl;
or pharmaceutically acceptable salts and solvates thereof.

According to specific embodiments, the present invention provides the compounds of examples as listed in the table below, and pharmaceutical acceptable salts and solvates thereof.

| **Example No.** | **Chemical Name** |
|---|---|
| Example 1 | (R)-2-fluoro-4-(3-(methyl(7H-pyrrolo[2,3-d]pyrimidin-4-yl)amino)pyrrolidin-1-yl)benzonitrile |
| Example 2 | 5-(3-(methyl(7H-pyrrolo[2,3-d]pyrimidin-4-yl)amino)pyrrolidin-1-yl)picolinonitrile |
| Example 3 | (R)-6-(3-(methyl(7H-pyrrolo[2,3-d]pyrimidin-4-yl)amino)pyrrolidin-1-yl)-N-(thiazol-2-yl)nicotinamide |
| Example 4 | 7-((1-(4-cyano-3-fluorophenyl)pyrrolidin-3-yl)amino)thieno[3,2-b]pyridine-6-carboxamide |
| Example 5 | methyl 2-(4-chloro-2-fluorophenyl)-2-(3-(methyl(7H-pyrrolo[2,3-d]pyrimidin-4-yl)amino)pyrrolidin-1-yl)acetate |
| Example 6 | 2-(4-chloro-2-fluorophenyl)-2-(3-(methyl(7H-pyrrolo[2,3-d]pyrimidin-4-yl)amino)pyrrolidin-1-yl)acetic acid |
| Example 7 | ethyl 7-((1-(4-cyano-3-fluorophenyl)pyrrolidin-3-yl)amino)thieno[3,2-b]pyridine-6-carboxylate |
| Example 8 | 7-((1-(4-cyano-3-fluorophenyl)pyrrolidin-3-yl)amino)thieno[3,2-b]pyridine-6-carboxylic acid |
| Example 9 | (R)-4-fluoro-2-(3-(methyl(7H-pyrrolo[2,3-d]pyrimidin-4-yl)amino)pyrrolidin-1-yl)benzonitrile |
| Example 10 | (R)-5-(3-(methyl(7H-pyrrolo[2,3-d]pyrimidin-4-yl)amino)pyrrolidin-1-yl)pyrimidine-2-carbonitrile |

The compounds of the invention, including all the compounds hereabove listed, can be prepared from readily available starting materials using general methods and procedures as described in the experimental part below or by using slightly modified processes readily available to those of ordinary skill in the art. Although a particular embodiment of the present invention may be shown or described herein, those skilled in the art will recognize that all embodiments or aspects of the present invention can be prepared using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

As herein described in details, the compounds of the invention are inhibitors of kinase activity, in particular. inhibiting JAK kinase activity for the treatment of JAK-dependent diseases.

In one aspect the invention provides compounds according to the invention , i.e. a compound of formula (I) or a pharmaceutical composition thereof, for use as a medicament, preferably for the prevention and /or treatment of respiratory and specifically pulmonary disease.

In a further aspect the invention provides the use of a compound (I), or a pharmaceutically acceptable salt thereof, in the manufacture of a medicament for the treatment of disorders associated with JAK mechanisms, particularly for the treatment of disorders such as respiratory and pulmonary diseases.

In particular the invention provides compounds of formula (I) for use in the prevention and /or treatment of pulmonary disease selected from the group consisting of asthma, chronic obstructive pulmonary disease COPD, idiopathic pulmonary fibrosis (IPF)acute lung injury and acute respiratory distress syndrome (ARDS).

Moreover the invention provides a method for the prevention and/or treatment of disorders associated with JAK mechanisms, said method comprising administering to a patient in need of such treatment a therapeutically effective amount of a compound of the invention.

In particular the invention provides methods for the prevention and/or treatment wherein the disorder is a respiratory disease selected from asthma, chronic obstructive pulmonary disease (COPD), idiopathic pulmonary fibrosis (IPF), acute lung injury and acute respiratory distress syndrome (ARDS).

Preferred is the use of the compounds of the invention for the prevention of the aforesaid disorders.

Equally preferred is the use of the compounds of the invention for the treatment of the aforesaid disorders.

Generally speaking, compounds which are JAK inhibitors may be useful in the treatment of many disorders associated with JAK enzyme mechanisms.

In one embodiment, the disorder that can be treated by the compound of the present invention is selected from the group consisting of asthma, chronic obstructive pulmonary disease (COPD) and interstitial lung disease such as idiopathic pulmonary fibrosis (IPF), acute lung injury and acute respiratory distress syndrome (ARDS).

In a further embodiment, the disorder is selected from asthma and chronic obstructive pulmonary disease (COPD).

The methods of treatment of the invention comprise administering an effective amount of a compound of formula (I) or a pharmaceutically acceptable salt thereof to a patient in need thereof. As used herein, " effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

The present invention is also directed to use of the compounds of the invention and their pharmaceutical compositions for various route of administration.

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the present invention may be formulated as injectable composition, for example to be injected intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

For the treatment of the diseases of the respiratory tract, the compounds according to the invention, as above said, may be administered by inhalation.

Inhalable preparations include inhalable powders, propellant-containing metering aerosols or propellant-free inhalable formulations and may be administered through a suitable inhalation device which may be respectively selected from dry powder inhaler, pressurized metered dosed inhaler, or a nebulizer.

For administration as a dry powder, single- or multi-dose inhalers known from the prior art may be utilized. In that case the powder may be filled in gelatine, plastic or other capsules, cartridges or blister packs or in a reservoir.

A diluent or carrier, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in the form of solutions or suspensions in an aqueous, alcoholic or hydroalcoholic medium and they may be delivered by jet or ultrasonic nebulizers known from the prior art or by soft-mist nebulizers such as Respimat^{®},a registered trademark of Boehringer Ingelheim Pharmaceuticals (Wachtel, H., Kattenbeck, S., Dunne, S. et al. Pulm Ther (2017) 3: 19.

The compounds of the invention, regardless of the route of administration, can be administered as the sole active agent or in combination (i.e. as co-therapeutic agents administered in fixed dose combination or in combined therapy of separately formulated active ingredients) with other pharmaceutical active ingredients.

The compounds of the invention can be administered as the sole active agent or in combination with other pharmaceutical active ingredients including those currently used in the treatment of respiratory disorders, and known to the skilled person, such as beta2-agonists, antimuscarinic agents, corticosteroids mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators).

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

A pharmaceutical composition comprising a compound of the invention suitable to be administered by inhalation is in various respirable forms, such as inhalable powders (DPI), propellant-containing metering aerosols (PMDI) or propellant-free inhalable formulations (e.g. UDV).

The invention is also directed to a device comprising the pharmaceutical composition comprising a compound according to the invention, which may be a single- or multi-dose dry powder inhaler, a metered dose inhaler and a nebulizer particularly soft mist nebulizer.

The invention is also directed to a kit comprising the pharmaceutical compositions of compounds of the invention alone or in combination with or in admixture with one or more pharmaceutically acceptable carriers and/or excipients and a device which may be a single- or multi-dose dry powder inhaler, a metered dose inhaler or a nebulizer.

The features of the invention will become apparent in the course of the following descriptions of exemplary embodiments which are given for illustration of the invention and are not intended to be limiting thereof.

### EXAMPLES

The following compounds of Example 1-10 were prepared and characterized as follows:

| **Example No.** | **Structure** | **Chemical Name** | **Biochemical Potency JAK 1 (pIC50)** | **Cell Based assay PBMC (IL-2 stimulated pSTAT5)** | **1H-NMR** | **LC-MS** |
|---|---|---|---|---|---|---|
| Example 1 | 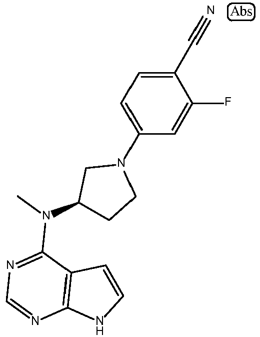 | (R)-2-fluoro-4-(3-(methyl(7H-pyrrolo[2,3-d]pyrimidin-4-yl)amino)pyrrolidin-1-yl)benzonitrile | 8.6 | 7.3 | ¹H-NMR (500 MHz, *dmso-d₆*) δ: 11.69 (bs, 1H); 8.14 (s, 1H); 7.57 (t, J = 8.5 Hz, 1H); 7.17 (d, J = 3.6 Hz, 1H); 6.64 (d, J = 3.5 Hz, 1H); 6.59 (dd, J = 13.0, 1.8 Hz, 1H); 6.53 (dd, J = 8.8, 1.9 Hz, 1H); 5.66-5.72 (m, 1H); 3.57-3.63 (m, 2H); 3.39-3.44 (m, 2H); 3.23 (s, 3H); 2.20-2.22 (m, 2H). | [MH]+ m/z 337.5 |
| Example 2 | 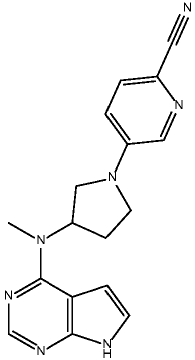 | 5-(3-(methyl(7H-pyrrolo[2,3-d]pyrimidin-4-yl)amino)pyrrolidin-1-yl)picolinonitrile | 7.9 | 6.6 | ¹H-NMR (500 MHz, *dmso-d₆*) δ: 11.69 (bs, 1H); 8.14 (s, 1H); 8.10 (d, J = 2.7 Hz, 1H); 7.73 (d, J = 8.3 Hz, 1H); 7.17 (t, J = 2.3 Hz, 1H); 7.02 (dd, J = 8.4, 2.7 Hz, 1H); 6.65 (d, J = 2.3 Hz, 1H); 1H); 5.70-5.75 (m, 1H); 3.63-3.67 (m, 2H); 3.43-3.48 (m, 2H); 3.24 (s, 3H); 2.26-2.29 (m, 2H). | [MH]+ m/z 320.3 |
| Example 3 | 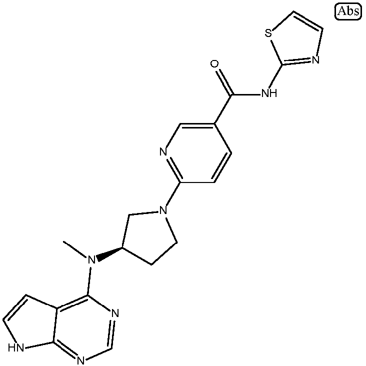 | (R)-6-(3-(methyl(7H-pyrrolo[2,3-d]pyrimidin-4-yl)amino)pyrrolidin-1-yl)-N-(thiazol-2-yl)nicotinamide | 7.7 | 6.3 | ¹H-NMR (500 MHz, *dmso-d₆*) δ: 12.3 (bs, 1H); 11.69 (bs, 1H); 8.87 (d, J = 2.3 Hz, 1H); 8.19 (dd, J = 9.0, 2.5 Hz, 1H); 8.15 (s, 1H); 7.52 (d, J = 3.4 Hz, 1H); 7.22 (d, J = 3.4 Hz, 1H); 7.17 (d, J = 3.4, 2.6 Hz, 1H); 6.61 (d, J = 9.0 Hz, 1H); 5.65-5.70 (m, 1H); 3.77-3.83 (m, 2H); 3.52-3.57 (m, 2H); 3.26 (s, 3H); 2.26-2.30 (m, 2H). | [MH]+ m/z 421.1 |
| Example 4 | 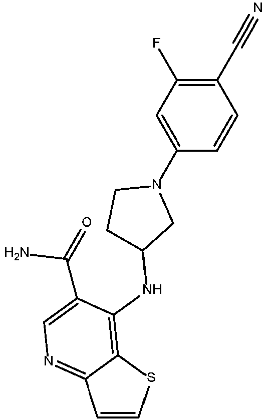 | 7-((1-(4-cyano-3-fluorophenyl)pyrrolidin-3-yl)amino)thieno[3,2-b]pyridine-6-carboxamide | 7 | 5.5 | ¹H-NMR (500 MHz, *dmso-d₆*) δ: 10.28 (d, J = 6.3 Hz, 1H); 8.73 (s, 1H); 8.23 (d, J= 5.5 Hz, 1H); 8.18 (bs, 1H); 7.76 (t, J = 8.3 Hz, 1H); 7.45 (bs, 1H); 7.44 (d, J = 5.5 Hz, 1H); 6.61 (dd, J = 13.4, 1.3 Hz, 1H); 6.52 (dd, J = 8.6, 1.2 Hz, 1H); 4.96-5.00 (m, 1H); 3.79 (dd, J = 10.4, 5.2 Hz, 1H); 3.49-3.54 (m, 1H); 3.47 (m, 2H); 2.43-2.47 (m, 1H); 2.11-2.17 (m, 1H). | [MH]+ m/z 382.2 |
| Example 5 | 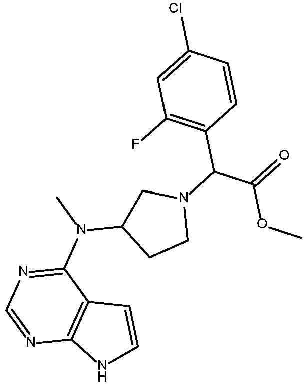 | methyl 2-(4-chloro-2-fluorophenyl)-2-(3-(methyl(7H-pyrrolo[2,3-d]pyrimidin-4-yl)amino)pyrrolidin-1-yl)acetate | 7.6 | not yet available | ¹H-NMR (600 MHz, *dmso-d₆*) δ: 11.60 (bs, 2H); 8.08 (s, 1H); 8.07 (s, 1H); 7.58 (t, J = 8.1 Hz, 1H); 7.55 (t, J = 8.1 Hz, 1H); 7.49 (t, J = 1.9 Hz, 1H); 7.48 (t, J = 1.9 Hz, 1H); 7.36-7.37 (m, 1H); 7.35-7.36 (m, 1H); 7.11-7.12 (m, 2H); 6.59 (dd, J = 3.5, 1.8 Hz, 1H); 6.57 (dd, J = 3.5, 1.8 Hz, 1H); 5.45-5.54 (m, 2H); 4.61 (s, 1H); 4.53 (s, 1H); 3.64 (s, 3H); 3.65 (s, 3H); 3.30 (s, 3H); 3.28 (s, 3H); 2.94-2.98 (m, 1H); 2.84 (dd, J = 9.6, 4.0 Hz, 1H); 2.73-2.76 (m, 1H); 2.67-2.70 (m, 2H); 2.57-2.60 (m, 1H); 2.45-2.43 (m, 2H); 2.12-2.19 (m, 2H); 1.75-1.87 (m, 2H). | [MH]+ m/z 418.1 |
| Example 6 | 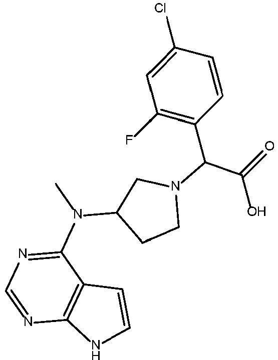 | 2-(4-chloro-2-fluorophenyl)-2-(3-(methyl(7H-pyrrolo[2,3-d]pyrimidin-4-yl)amino)pyrrolidin-1-yl)acetic acid | 5.3 | not yet available | ¹H-NMR (500 MHz, *dmso-d₆*) δ: 12.14 (bs, 2H); 8.22 (s, 1H); 8.20 (s, 1H); 7.64-7.72 (m, 2H); 7.57-7.61 (m, 2H); 7.41-7.45 (m, 2H); 7.30 (bs, 2H); 6.78 (bs, 1H); 6.76 (bs, 1H); 5.33 (bs, 2H); 5.13-5.22 (m, 2H); 3.40 (s, 3H); 3.38 (s, 3H); 3.08-3.19 (m, 2H); 2.81-2.92 (m, 2H); 2.24-2.33 (m, 2H); 2.04-2.11 (m, 2H). | [MH]+ m/z 404.1 |
| Example 7 | 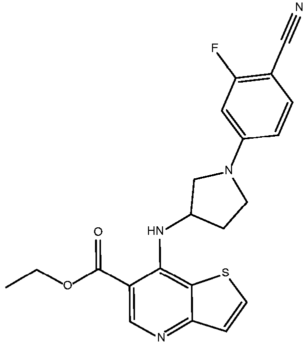 | ethyl 7-((1-(4-cyano-3-fluorophenyl)pyrrolidin-3-yl)amino)thieno[3,2-b]pyridine-6-carboxylate | 6 | 5.6 | ¹H-NMR (500 MHz, *dmso-d₆*) δ: 9.13 (d, J = 8.1 Hz, 1H); 8.84 (s, 1H); 8.26 (d, J= 5.3 Hz, 1H); 7.57 (t, J = 8.3 Hz, 1H); 7.49 (d, J = 5.3 Hz, 1H); 6.62 (dd, J = 13.4, 1.3 Hz, 1H); 6.54 (dd, J = 8.6, 1.2 Hz, 1H); 5.03-5.06 (m, 1H); 3.81 (dd, J = 11.0, 5.4 Hz, 1H); 3.50-3.57 (m, 2H); 3.47 (dd, J = 11.1, 3.0 Hz, 1H); 2.50 (m, 1H); 2.17-2.23 (m, 1H); 1.31 (t, J = 7.4 Hz, 3H). | [MH]+ m/z 411.2 |
| Example 8 | 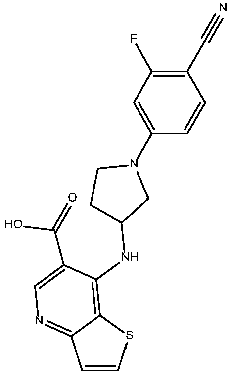 | 7-((1-(4-cyano-3-fluorophenyl)pyrrolidin-3-yl)amino)thieno[3,2-b]pyridine-6-carboxylic acid | 5.3 | 4.5 | ¹H-NMR (500 MHz, *dmso-d₆*) δ: 8.84 (s, 1H); 8.27 (d, J= 5.3 Hz, 1H); 7.56 (t, J = 8.3 Hz, 1H); 7.47 (d, J = 5.3 Hz, 1H); 6.62 (dd, J = 13.4, 1.3 Hz, 1H); 6.53 (dd, J = 8.6, 1.2 Hz, 1H); 4.99-5.04 (m, 1H); 4.29 8 (q, J = 7.4 Hz, 1H); 3.82 (dd, J = 11.0, 5.4 Hz, 1H); 3.52-3.55 (m, 2H); 3.32 3.47 (dd, J = 11.1, 3.0 Hz, 1H); 2.45-4.48 (m, 1H); 2.17-2.15-2.20 (m, 1H). | [MH]+ m/z 383.2 |
| Example 9 | 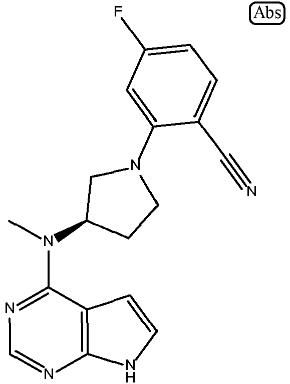 | (R)-4-fluoro-2-(3-(methyl(7H-pyrrolo[2,3-d]pyrimidin-4-yl)amino)pyrrolidin-1-yl)benzonitrile | 7.6 | 6.4 | ¹H-NMR (500 MHz, *dmso-d₆*) δ: 11.69 (bs, 1H); 8.14 (s, 1H); 7.61 (dd, J = 8.6, 6.7 Hz, 1H); 7.17 (d, J = 3.6 Hz, 1H); 6.65 (d, J = 3.5 Hz, 1H); 6.65 (dd, J = 12.8, 2.2 Hz, 1H); 6.59 (td, J = 8.1, 2.1 Hz, 1H); 5.59-5.65 (m, 1H); 3.73-3.80 (m, 2H); 3.62-3.70 (m, 2H); 3.27 (s, 3H); 2.19-2.32 (m, 2H). | [MH]+ m/z 337.5 |
| Example 10 | 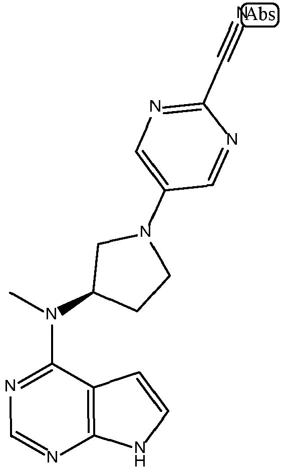 | (R)-5-(3-(methyl(7H-pyrrolo[2,3-d]pyrimidin-4-yl)amino)pyrrolidin-1-yl)pyrimidine-2-carbonitrile | 7.9 | 6.7 | ¹H-NMR (500 MHz, *dmso-d₆*) δ: 11.69 (bs, 1H); 8.27 (s, 2H); 8.14 (s, 1H); 7.17 (t, J = 2.9 Hz, 1H); 6.65 (dd, J = 3.2, 1.6 Hz, 1H); 1H); 5.71-5.77 (m, 1H); 3.68-3.73 (m, 2H); 3.46-3.51 (m, 2H); 3.24 (s, 3H); 2.22-2.30 (m, 2H). | [MH]+ m/z 321.1 |

The compound of Example 2 was prepared according to the following scheme:

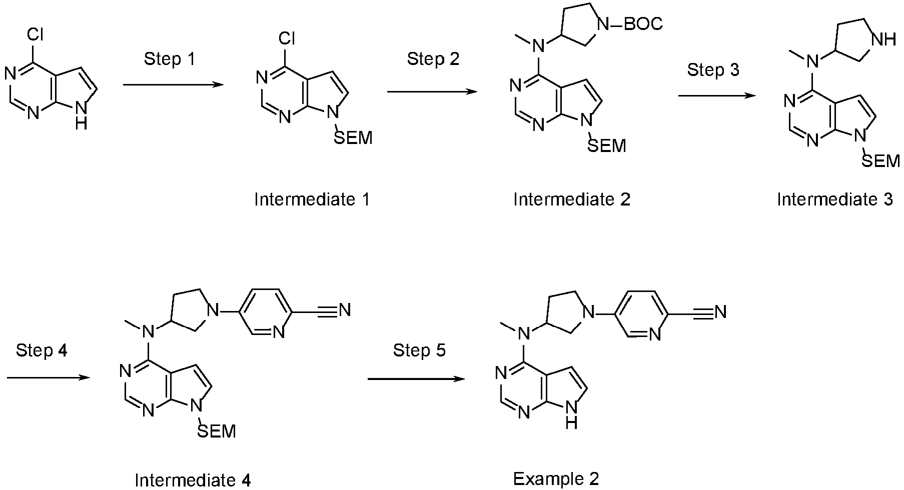

### Step 1

### Intermediate 1: (2-[(4-chloropyrrolo[2,3-d]pyrimidin-7-yl)methoxy]ethyl-trimethylsilane)

To a solution of 4-chloro-7H-pyrrolo[2,3-d]pyrimidine (5 g, 33 mmol) in tetrahydrofurane (50 ml) was added NaH (60% in paraffin liquid) (2 g, 49 mmol) at 0°C. The reaction mixture was stirred at 0°C for 1h before addition of the 2-(chloromethoxyethyl)trimethyl silane (16 ml, 88 mmol), then 3h at room temperature. Reaction was quenched by the addition of water (50 ml) and extracted with ethyl acetate (100 ml). Organic layer was dried over Na₂SO₄ and evaporated in vacuum to give crude product that was purified by cromatography on silica gel to give the title product (8.4 g). ES⁺ 284.5 [M+H]⁺

### Step 2

### Intermediate 2: (tert-butyl 3-[methyl-[7-(2-trimethylsilylethoxymethyl)pyrrolo[2,3-d]pyrimidin-4-yl]amino]pyrrolidine-1-carboxylate)

To a suspension of Intermediate 1 (1.1 g, 4.0 mmol) in dry dimethylformammide (10 mL), tert-butyl 3-(methylamino) pyrrolidine-1-carboxylate (800 mg, 2.8 mmol) and K₂CO₃ (1.1 g, 8 mmol) were added. Reaction mixture was heated in a microwave reactor at temperature up to 140 °C for several hours. Reaction mixture was diluted with water (60 mL) and extracted with ethyl acetate (3x60 mL). Organic layer was washed with aqueous saturated NaHCO₃ (2x50 mL), dried over Na₂SO₄ and evaporated to give crude product. Crude product was purified on silica gel to afford the title compound (480 mg). ES⁺ 448.7 [M+H]⁺

### Step 3

### Intermediate 3 : (N-methyl-N-pyrrolidin-3-yl-7-(2-trimethylsilylethoxymethyl)pyrrolo[2,3-d]pyrimidin-4-amine)

Intermediate 2 (480 mg, 1.07 mmol) was dissolved in 4M HCl solution in dioxane (5 mL). The solvent was evaporated under vacuum and the crude purified on SCX (strong cathion exchange) column by eluting with methanol- ammonia in methanol 2 M, to afford the title product (320 mg). ES⁺ 348.3 [M+H]⁺

### Step 4

### Intermediate 4 (5-[3-[methyl-[7-(2-trimethylsilylethoxymethyl)pyrrolo[2,3-d]pyrimidin-4-yl]amino]pyrrolidin-1-yl]pyridine-2-carbonitrile):

Under nitrogen atmosphere, to a reaction mixture of 5-bromopyrimidine-2-carbonitrile (62 mg, 0.34 mmol), Cs₂CO₃ (169 mg, 0.52 mmol), RuPhos (2-Dicyclohexylphosphino-2',6'-diisopropoxybiphenyl) (6 mg, 0.013 mmol) and Pd₂(dba)₃ (Bis(dibenzylideneacetone)palladium(0)) (12 mg, 0.013 mmol), a solution of Intermediate 3 (90 mg, 0.26 mmol) in dry 1,4-dioxane (2 mL) was added dropwise. The resulting solution was heated to 110 °C for 20h. Reaction mixture was cooled to room temperature diluted with water and extracted with ethyl acetate. Combined organic layers was washed with saturated aqueous NaCl, dried over anhydrous Na₂SO₄ and concentrated in vacuo. Crude product was purified on silica gel to yield (26 mg) the title compound. ES⁺ 450.8 [M+H]⁺

### Step 5

### Example 2: (5-[3-[methyl(7H-pyrrolo[2,3-d]pyrimidin-4-yl)amino]pyrrolidin-1-yl]pyridine-2-carbonitrile):

Intermediate 4 (25 mg, 0.05 mmol) was dissolved in dichloromethane (2 mL), then trifluoroacetic acid (1mL, 13.5 mmol) added and reaction mixture stirred at rt for 1.5 hours. The solvent was evaporated and ammonia 7 M in methanol (1 mL) were added to the residue, then the reaction mixture heated at 60 °C for 1 hour. After solvent evaporation, the crude material was purified by SCX (strong cathion exchange) column by eluting with methanol- ammonia in methanol 2 M. ES⁺ 320.2 [M+H]⁺

The compounds of example 1 and 3-10 were prepared in a similar manner to Example 2, following the same synthetic sequence; or, where modification of reaction conditions reactants or solvents were used, they can be readily determined by those skilled in the art by routine optimization procedures.

### Biochemical Potency JAK1 (Data displayed into the table as pIC50)

The objective of this study was to assess the activity of novel JAK inhibitors measuring the capability of compounds to inhibit JAK1 kinase activity in a biochemical time-resolved fluorescence resonance energy transfer (TR-FRET) LANCE assay. In LANCE Ultra kinase assay in the presence of JAK1 kinase and ATP (corresponding to Km), the ULight peptide substrate (LANCE Ulight-JAK-1 (Tyr1023) Peptide, Perkin Elmer, TRF0121) was phosphorylated. It was then captured by a Eu-anti-phospho-substrate antibody (LANCE Eu-W1024 Anti-phosphotyrosine (PT66), Perkin Elmer, AD0069), which brought the Eu-chelate donor and ULight acceptor dyes into close proximity. Upon excitation at 320 nm, the Eu-chelate transfers its energy to the ULight dye, resulting in a fluorescent light emission at 665 nm. Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.5, Hill Slope range 0.5 to 5, S:B > 2.

In addition to enzymatic potency, the effects of the inhibitors against JAK1/JAK3 activity in a cellular assay was characterized against IL-2 induced phosphorylation of STAT5 level in human peripheral blood mononuclear cells (PBMCs).

### Cell Based assay PBMC (IL-2 stimulated pSTAT5) (Data displayed into the table as pIC50)

PBMC have been isolated from human healthy volunteers. Cells were seeded in wells and treated with compounds and rh IL-2. After 30 min incubation cells were lysed and pSTAT5 determined by PathScan phospho-stat5 (Tyr694) ELISA (Cell signaling). Inhibitors were tested at 11 consecutive 5-fold dilutions starting from 30 µM (30 µM - 3 pM) in duplicate. Calculation of IC50 data, curves and QC analysis were made using Excel tools and GraphPadPrism software. QC criteria parameters: Z' ≥ 0.35, Hill Slope range 0.5 to 5, S:B > 2.

### NMR spectra

NMR spectra were recorded on a Bruker Avance III 600 (5 mm RT inverse probehead), Bruker DRX 500, Bruker Avance AV 400 (5 mm RT direct probehead) and Bruker DPX 300 spectrometers using standard Bruker pulse sequences. DMSO-d₆ or CDCl3 were used as solvents and TMS as the internal standard unless in the latter case where solvent residual peak was used. All experiments were recorded at 25 °C, unless stated differently.

LC-MS spectra were recorded on Acquity UPLC coupled with SQD mass spectrometer. **Chromatographic Columns:** Acquity UPLC BEH C18 (50mm x 2.1mm i.d., 1.7µm packing diameter), or Acquity UPLC BEH C18 (50mm x 2.1mm i.d., 1.7µm packing diameter), column temperature 40 °C. Mobile phase: A = 0.1% v/v solution of formic acid in water, B = 0.1% v/v solution of formic acid in acetonitrile or A = 10 mM aqueous solution of NH4HCO3 (adjusted to pH 10 with ammonia) and B = Acetonitrile. Analytical samples were dissolved in mixture of water:acetonitrile (1:1). If necessery about 10 % of dmso was used in order to improve solubility.

Where a numerical limit or range is stated herein, the endpoints are included. Also, all values and subranges within a numerical limit or range are specifically included as if explicitly written out.

As used herein the words "a" and "an" and the like carry the meaning of "one or more."

Obviously, numerous modifications and variations of the present invention are possible in light of the above teachings. It is therefore to be understood that, within the scope of the appended claims, the invention may be practiced otherwise than as specifically described herein.

## Claims

1. 3-aminopyrrolidine derivatives of formula I 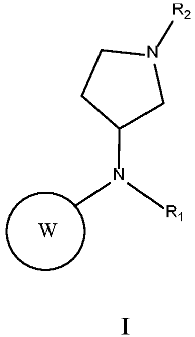 wherein,
R₁ is H or methyl;
R₂ is selected from a phenyl, phenylmethyl, pyridinyl and pyrimidinyl group substituted by one or more group selected from the group -CN, F, Cl, (thiazol-2-yl)aminocarbonyl, (methoxy)carbonyl, (hydroxy)carbonyl;
W is a bicyclic heteroaryl selected from J1 or J2: 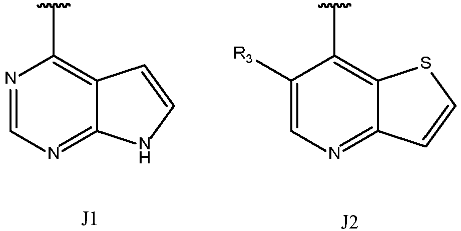 wherein,
R₃ is (ethoxy)carbonyl, (hydroxy)carbonyl or (amino)carbonyl;
single enantiomers, diastereoisomers and mixtures thereof
or a pharmaceutically acceptable salt or solvate thereof.

2. A compound according to claim 1 selected from:
(R)-2-fluoro-4-(3-(methyl(7H-pyrrolo[2,3-d]pyrimidin-4-yl)amino)pyrrolidin-1-yl)benzonitrile;
5-(3-(methyl(7H-pyrrolo[2,3-d]pyrimidin-4-yl)amino)pyrrolidin-1-yl)picolinonitrile
(R)-6-(3-(methyl(7H-pyrrolo[2,3-d]pyrimidin-4-yl)amino)pyrrolidin-1-yl)-N-(thiazol-2-yl)nicotinamide;
7-((1-(4-cyano-3-fluorophenyl)pyrrolidin-3-yl)amino)thieno[3,2-b]pyridine-6-carboxamide;
methyl 2-(4-chloro-2-fluorophenyl)-2-(3-(methyl(7H-pyrrolo[2,3-d]pyrimidin-4-yl)amino)pyrrolidin-1-yl)acetate;
2-(4-chloro-2-fluorophenyl)-2-(3-(methyl(7H-pyrrolo[2,3-d]pyrimidin-4-yl)amino)pyrrolidin-1-yl)acetic acid;
ethyl 7-((1-(4-cyano-3-fluorophenyl)pyrrolidin-3-yl)amino)thieno[3,2-b]pyridine-6-carboxylate;
7-((1-(4-cyano-3-fluorophenyl)pyrrolidin-3-yl)amino)thieno[3,2-b]pyridine-6-carboxylic acid;
(R)-4-fluoro-2-(3-(methyl(7H-pyrrolo[2,3-d]pyrimidin-4-yl)amino)pyrrolidin-1-yl)benzonitrile;
(R)-5-(3-(methyl(7H-pyrrolo[2,3-d]pyrimidin-4-yl)amino)pyrrolidin-1-yl)pyrimidine-2-carbonitrile;
single enantiomers, diastereoisomers and mixtures thereof
or a pharmaceutically acceptable salt or solvate thereof.

3. A pharmaceutical composition comprising a compound as defined in any one of claims 1 to 2, or a pharmaceutically acceptable salt thereof, in admixture with one or more pharmaceutically acceptable carrier or excipient.

4. A pharmaceutical composition according to claim 3 suitable to be administered by inhalation, selected from inhalable powders, propellant-containing metering aerosols or propellant-free inhalable formulations.

5. A device comprising the pharmaceutical composition according to claim 4, which may be a single- or multi-dose dry powder inhaler, a metered dose inhaler or a soft mist nebulizer.

6. A compound or a pharmaceutical composition according to any one of claims 1 to 4 for use as a medicament.

7. A compound or a pharmaceutical composition for use according to claim 6 in the prevention and /or treatment of a pulmonary disease selected from the group consisting of asthma, chronic obstructive pulmonary disease COPD, idiopathic pulmonary fibrosis (IPF), acute lung injury and acute respiratory distress syndrome (ARDS).

8. A combination of a compound as defined in any one of the claims 1 to 2 with one or more active ingredients selected from the classes currently used in the treatment of respiratory disorders, and known to the skilled person, such as beta2-agonists, antimuscarinic agents, corticosteroids, mitogen-activated kinases (P38 MAP kinases) inhibitors, nuclear factor kappa-B kinase subunit beta inhibitors (IKK2), human neutrophil elastase (HNE) inhibitors, phosphodiesterase 4 (PDE4) inhibitors, leukotriene modulators, non-steroidal anti-inflammatory agents (NSAIDs) and mucus regulators.

## Patentansprüche

1. 3-Aminopyrrolidinderivate der Formel I 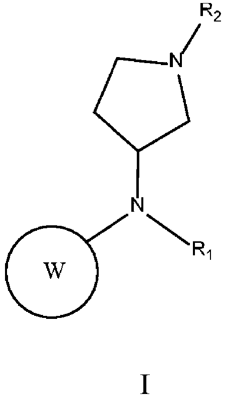 wobei,
R₁ H oder Methyl ist;
R₂ aus einer Phenyl-, Phenylmethyl-, Pyridinyl- und Pyrimidinylgruppe ausgewählt ist, substituiert durch eine oder mehrere Gruppen, die aus der Gruppe -CN, F, Cl, (Thiazol-2-yl)aminocarbonyl, (Methoxy)carbonyl, (Hydroxy)carbonyl ausgewählt sind;
W ein bicyclisches Heteroaryl ist, das aus J1 oder J2 ausgewählt ist: 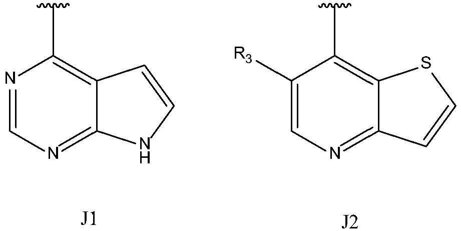 wobei,
R₃ (Ethoxy)carbonyl, (Hydroxy)carbonyl oder (Amino)carbonyl ist;
einzelne Enantiomere, Diastereoisomere und Mischungen davon
oder ein pharmazeutisch annehmbares Salz oder Solvat davon.

2. Verbindung nach Anspruch 1, ausgewählt aus:
(R)-2-fluor-4-(3-(methyl(7H-pyrrolo[2,3-d]pyrimidin-4-yl)amino)pyrrolidin-1-yl)benzonitril;
5-(3-(Methyl(7H-pyrrolo[2,3-d]pyrimidin-4-yl)amino)pyrrolidin-1-yl)picolinonitril
(R)-6-(3-(Methyl(7H-pyrrolo[2,3-d]pyrimidin-4-yl)amino)pyrrolidin-1-yl)-N-(thiazol-2-yl)nicotinamid;
7-((1-(4-Cyano-3-fluorphenyl)pyrrolidin-3-yl)amino)thieno[3,2-b]pyridin-6-carboxamid;
Methyl-2-(4-chlor-2-fluorphenyl)-2-(3-(methyl(7H-pyrrolo[2,3-d]pyrimidin-4-yl)amino)pyrrolidin-1-yl)acetat;
2-(4-Chlor-2-fluorphenyl)-2-(3-(methyl(7H-pyrrolo[2,3-d]pyrimidin-4-yl)amino)pyrrolidin-1-yl)essigsäure;
Ethyl-7-((1-(4-cyano-3-fluorphenyl)pyrrolidin-3-yl)amino)thieno[3,2-b]pyridin-6-carboxylat;
7-((1-(4-cyano-3-fluorphenyl)pyrrolidin-3-yl)amino)thieno[3,2-b]pyridin-6-carbonsäure;
(R)-4-Fluor-2-(3-(methyl(7H-pyrrolo[2,3-d]pyrimidin-4-yl)amino)pyrrolidin-1-yl)benzonitril;
(R)-5-(3-(Methyl(7H-pyrrolo[2,3-d]pyrimidin-4-yl)amino)pyrrolidin-1-yl)pyrimidin-2-carbonitril;
einzelnen Enantiomeren, Diastereoisomeren und Mischungen davon
oder einem pharmazeutisch annehmbaren Salz oder Solvat davon.

3. Pharmazeutische Zusammensetzung, umfassend eine Verbindung nach einem der Ansprüche 1 bis 2 oder ein pharmazeutisch annehmbares Salz davon, unter Beigabe eines oder mehrerer pharmazeutisch annehmbarer Träger oder Hilfsstoffe.

4. Pharmazeutische Zusammensetzung nach Anspruch 3, die geeignet ist, um durch Inhalation verabreicht zu werden, ausgewählt aus inhalierbaren Pulvern, treibmittelhaltigen Dosieraerosolen oder treibmittelfreien inhalierbaren Formulierungen.

5. Vorrichtung, umfassend die pharmazeutische Zusammensetzung nach Anspruch 4, die ein Einzel- oder Mehrfachdosis-Trockenpulverinhalator, ein Dosierinhalator oder ein Soft-Mist-Inhalator sein kann.

6. Verbindung oder pharmazeutische Zusammensetzung nach einem der Ansprüche 1 bis 4 zur Verwendung als ein Arzneimittel.

7. Verbindung oder pharmazeutische Zusammensetzung zur Verwendung nach Anspruch 6 bei der Prävention und/oder Behandlung einer pulmonalen Erkrankung, die aus der Gruppe bestehend aus Asthma, chronisch obstruktiver pulmonaler Erkrankung COPD, idiopathischer pulmonaler Fibrose (IPF), akuter Lungenverletzung und akutem Atemnotsyndrom (ARDS) ausgewählt ist.

8. Kombination einer Verbindung nach einem der Ansprüche 1 bis 2 mit einem oder mehreren Wirkstoffen, die aus den Klassen ausgewählt sind, die aktuell bei der Behandlung von Atemstörungen verwendet werden und der Fachperson bekannt sind, wie Beta2-Agonisten, Antimuskarinika, Kortikosteroide, Inhibitoren von mitogenaktivierten Kinasen (P38 MAP-Kinasen), Inhibitoren von Nuklearfaktor-kappa-B-Kinase-Untereinheit Beta (IKK2), Inhibitoren von humaner neutrophiler Elastase (HNE), Inhibitoren von Phosphodiesterase-4 (PDE4), Leukotrienmodulatoren, nicht-steroidale Antiphlogistika (NSAR) und Schleimregulatoren.

## Revendications

1. Dérivés de 3-aminopyrrolidine de formule I 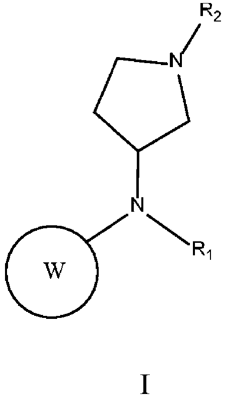 où,
R₁ est H ou méthyle ;
R₂ est choisi dans un groupe phényle, phénylméthyle, pyridinyle et pyrimidinyle substitué par un ou plusieurs groupes choisis dans le groupe -CN, F, CI, (thiazol-2-yl)aminocarbonyle, (méthoxy)carbonyle, (hydroxy)carbonyle ;
W est un hétéroaryle bicyclique choisi parmi J1 ou J2 : 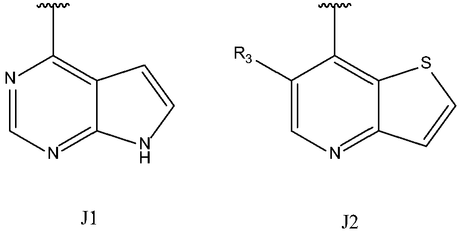 où,
R₃ est (éthoxy)carbonyle, (hydroxy)carbonyle ou (amino)carbonyle ;
énantiomères simples, diastéréoisomères et mélanges de ceux-ci
ou sel ou solvate acceptable sur le plan pharmaceutique de ceux-ci.

2. Composé selon la revendication 1 choisi parmi :
(R)-2-fluoro-4-(3-(méthyl(7H-pyrrolo[2,3-d]pyrimidin-4-yl)amino)pyrrolidin-1-yl)benzonitrile ;
5-(3-(méthyl(7H-pyrrolo[2,3-d]pyrimidin-4-yl)amino)pyrrolidin-1-yl)picolinonitrile
(R)-6-(3-(méthyl(7H-pyrrolo[2,3-d]pyrimidin-4-yl)amino)pyrrolidin-1-yl)-N-(thiazol-2-yl)nicotinamide ;
7-((1-(4-cyano-3-fluorophényl)pyrrolidin-3-yl)amino)thiéno[3,2-b]pyridine-6-carboxamide ;
2-(4-chloro-2-fluorophényl)-2-(3-(méthyl(7H-pyrrolo[2,3-d]pyrimidin-4-yl)amino)pyrrolidin-1-yl)acétate de méthyle ;
acide 2-(4-chloro-2-fluorophényl)-2-(3-(méthyl(7H-pyrrolo[2,3-d]pyrimidin-4-yl)amino)pyrrolidin-1-yl)acétique ;
7-((1-(4-cyano-3-fluorophényl)pyrrolidin-3-yl)amino)thiéno[3,2-b]pyridine-6-carboxylate d'éthyle ;
acide 7-((1-(4-cyano-3-fluorophényl)pyrrolidin-3-yl)amino)thiéno[3,2-b]pyridine-6-carboxylique ;
(R)-4-fluoro-2-(3-(méthyl(7H-pyrrolo[2,3-d]pyrimidin-4-yl)amino)pyrrolidin-1-yl)benzonitrile ;
(R)-5-(3-(méthyl(7H-pyrrolo[2,3-d]pyrimidin-4-yl)amino)pyrrolidin-1-yl)pyrimidine-2-carbonitrile ;
énantiomères simples, diastéréoisomères et mélanges de ceux-ci
ou sel ou solvate acceptable sur le plan pharmaceutique de ceux-ci.

3. Composition pharmaceutique comprenant un composé tel que défini selon l'une quelconque des revendications 1 à 2, ou un sel acceptable sur le plan pharmaceutique de celui-ci, en mélange avec un ou plusieurs transporteurs ou excipients acceptables sur le plan pharmaceutique.

4. Composition pharmaceutique selon la revendication 3, apte à être administrée par inhalation, choisie parmi des poudres inhalables, aérosols-doseurs contenant un agent propulseur ou formulations inhalables sans agent propulseur.

5. Dispositif comprenant la composition pharmaceutique selon la revendication 4, qui peut être un inhalateur de poudre sèche à dose unique ou à doses multiples, un aérosol-doseur ou un nébuliseur à brouillard doux.

6. Composé ou composition pharmaceutique selon l'une quelconque des revendications 1 à 4, pour une utilisation en tant que médicament.

7. Composé ou composition pharmaceutique pour une utilisation selon la revendication 6, dans la prévention et/ou le traitement d'une maladie pulmonaire choisie dans le groupe constitué d'asthme, broncho-pneumopathie chronique obstructive BPCO, fibrose pulmonaire idiopathique (FPI), lésion pulmonaire aiguë et syndrome de détresse respiratoire aiguë (SDRA).

8. Combinaison d'un composé défini selon l'une quelconque des revendications 1 à 2 avec un ou plusieurs ingrédients actifs choisis parmi les classes actuellement utilisées pour le traitement de troubles respiratoires et connus de la personne du métier, tels que bêta-2-agonistes, agents antimuscariniques, corticostéroïdes, inhibiteurs de kinases activées par mitogène (MAP-kinases P38), inhibiteurs de sous-unité bêta de kinase du facteur nucléaire kappa-B (IKK2), inhibiteurs d'élastase neutrophile humaine (HNE), inhibiteurs de phosphodiestérase 4 (PDE4), modulateurs de leucotriènes, anti-inflammatoires non stéroïdiens (AINS) et régulateurs de mucus.
